(19) Europäisches Patentamt
European Patent Office
Office européen des brevets

(11) Veröffentlichungsnummer: **0 245 596**
**A1**

(12) **EUROPÄISCHE PATENTANMELDUNG**

(21) Anmeldenummer: **87102799.1**

(51) Int. Cl.⁴: **G01N 11/14**

(22) Anmeldetag: **27.02.87**

(30) Priorität: **02.05.86 DE 3614995**

(43) Veröffentlichungstag der Anmeldung: **19.11.87 Patentblatt 87/47**

(84) Benannte Vertragsstaaten: **AT BE DE FR GB IT NL SE**

(71) Anmelder: **HÜLS AKTIENGESELLSCHAFT**
**Patentabteilung / PB 15 - Postfach 13 20**
**D-4370 Marl 1(DE)**

(72) Erfinder: **Bollenrath, Franz-Michael, Dr.**
**Lipper Weg 197**
**D-4370 Marl(DE)**
Erfinder: **Bartmann, Martin, Dr.**
**Burgstrasse 35**
**D-4350 Recklinghausen(DE)**

# (54) Verfahren zur Bestimmung des J-Wertes bei der oxidativen Kupplungsreaktion von Phenolen zu Polyphenylenethern.

(57) Die Erfindung betrifft ein Verfahren zur kontinuierlichen und schnellen Bestimmung des J-Wertes bei der oxidativen Kupplungsreaktion von Phenolen zu Polyphenylenethern, wobei man dem Reaktionsansatz Lösung einer definierten Temperatur entnimmt, die Viskosität in einem Couette-Viskosimeter mißt und den J-Wert mit Hilfe einer Eichkurve oder auf sonstige Weise bestimmt.

N2
7
N2
6
O2
8
LÖSUNG
2
REAKTOR
1
FC
9
O2R
M
TCR
PRODUKT
10
3
4
5

Fig. 1

EP 0 245 596 A1

# Verfahren zur Bestimmung des J-Wertes bei der oxidativen Kupplungsreaktion von Phenolen zu Polyphenylenethern

Die Erfindung betrifft ein Verfahren, um auf einfache Weise den J-Wert von Polyphenylenethern (PPE) während der oxidativen Kupplungsreaktion zu bestimmen. Der J-Wert stellt ein Maß für das Molekulargewicht des Polymeren dar.

Bekanntlich verändert sich der J-Wert zu Beginn der oxidativen Kupplungsreaktion nur wenig. Erst im letzten Drittel des Reaktionsablaufes steigt der J-Wert exponentiell an. In der Praxis ist man daran interessiert, ein PPE mit möglichst konstantem Molekulargewicht herzustellen. Gewünscht sind ferner PPE mit einem bestimmten J-Wert,da PPE mit niedrigerem oder höherem J-Wert anwendungstechnische Nachteile haben.

Dies setzt voraus, den J-Wert bei der oxidativen Kupplungsreaktion mit hinreichender Genauigkeit und Schnelligkeit zu bestimmen, um die Reaktion zum gewünschten Zeitpunkt abstoppen zu können. In der Praxis bereitet dies jedoch Probleme.

Mißt man die zu-und abgeführte Sauerstoffmenge, was mit hinreichender Genauigkeit prinzipiell nur mit Schwierigkeiten möglich wäre, so ist zu berücksichtigen, daß diese nur ein sehr unzureichendes Maß für den Reaktionsablauf darstellt. Spektroskopische Verfolgungen des Reaktionsverlaufes scheiden aus, da in Lösungen gearbeitet wird, die Katalysatoren und stark gefärbte Verunreinigungen enthalten. Um eine gezielte Abstoppung der PPE-Polykondensation durchzuführen, ist es erforderlich, die Messung innerhalb von weniger als einer Minute abschließen zu können.

Im Prinzip ist es bekannt, Viskosimeter zur Messung des J-Wertes von Polymerlösungen einzusetzen. Die DIN 53 728 sieht vor, daß man das Lösemittel abdampft, eine definierte verdünnte Lösung herstellt und sodann die Viskosität in einem Ubbelohde-Viskosimeter nach DIN 51 562 bestimmt. Ein solches Verfahren ist zeitlich sehr aufwendig und daher im vorliegenden Fall nicht geeignet. Es ist auch bekannt, Viskosimeter im Durchlauf zu betreiben und sie zur Verfolgung von Prozessen in chemischen Anlagen einzusetzen. Doch erscheint es aus folgenden Gründen als problematisch, diese Geräte bei der oxidativen Kupplungsreaktion von Phenolen einzusetzen:

1. Zu Beginn der Reaktion ist die Viskosität der Reaktionslösung außergewöhnlich niedrig; sie beträgt nur 1 - 6 mPa s und läßt sich mit den üblichen Geräten gar nicht erfassen.

2. Eine Reihe von Viskosimetern erlaubt lediglich die Messung speziell vorbereiteter Proben.

3. PPE scheiden sich stets in geringfügigem Maße an der Wand des Meßinstrumentes ab und beeinflussen damit die Messungen erheblich. Rohrrheometer sind daher grundsätzlich ungeeignet.

Ein Verfahren zur Bestimmung der Viskosität von PPE während der oxidativen Kupplungsreaktion ist aus der EP-PS 0 144 106 bekannt. Dieses Verfahren besteht darin, daß man dem Reaktor diskontinuierlich Proben entnimmt und durch Zugabe einer Flüssigkeit die Polymerisation abstoppt. Sobald man eine klare und homogene Lösung erhalten hat, kann die Durchlaufzeit der Lösung durch ein Kapillarrohrviskosimeter gemessen und aus dem Vergleich mit Standardwerten die Viskosität und daraus das Molekulargewicht ermittelt werden. Nachteilig ist, daß diese Methode grundsätzlich nur diskontinuierlich durchführbar ist. Während des schnellen Anstiegs der Viskosität in der entscheidenden Endphase der oxidativen Kupplung ist man nicht in der Lage, den augenblicklichen J-Wert der Reaktionslösung anzugeben.

Es wurde jetzt ein Verfahren gefunden, mit dem sich die geschilderten Nachteile vermeiden lassen. Dieses Verfahren besteht darin, daß man eine Lösung aus dem PPE-Reaktor mit einer definierten Temperatur durch das vorzugsweise thermostatisierte Couette-Viskosimeter fließen läßt, die Viskosität mißt und mit Hilfe einer Eichkurve oder auf sonstige Weise den J-Wert bestimmt. Anschließend kann die Lösung wieder in den Reaktor zurückgeführt werden. Gegebenenfall ist das Viskosimeter nach jedem Produktionsansatz mit dem Lösemittel zu spülen.

Obwohl die beschriebene Methode nicht der DIN 53 728 entspricht, hat sie sich überraschenderweise als recht praktikabel erwiesen.

Zwar liefert das Verfahren zu Beginn der Polykondensation nur grobe Werte, in dem entscheidenden Bereich höherer J-Werte von etwa 14 bis 30 m Pa s ist das Verfahren jedoch sehr genau. Es wurde festgestellt, daß die Zeit bis zum Beginn des exponentiellen Viskositätsanstieges in einem Zusammenhang mit dem erreichbaren J-Wert steht.

Im Prinzip ist das vorliegende Verfahren an ein bestimmtes Lösemittel und an eine bestimmte Konzentration des Polymeren im Lösemittel gebunden. Mit Hilfe mathematischer Beziehungen oder auch auf empirischen Wege durch Aufnahme von Eichkurven ist es möglich, auch bei vom Standard abweichenden Konzentrationen und Lösemitteln zu arbeiten.

Das beschriebene Verfahren ermöglicht es insbesondere, in den vorhandenen Lösungen unmittelbar die Viskosität zu messen und damit den momentanen J-Wert zu bestimmen. Die zeitliche Verzögerung, mit der die Messung erfolgt, liegt bei etwa 10 Sekunden und hängt von der mittleren Fließgeschwindigkeit und der Länge der Zulaufstrecke zum Rheometer ab. Vorteilhafterweise sorgt man dafür, daß diese Zulaufstrecke sehr kurz ist.

Im folgenden wird die Durchführung des Verfahrens näher beschrieben:

Die oxidative Kupplungsreaktion von diorthosubstituierten und bestimmten sterisch gehinderten monoorthosubstituierten Phenolen zu Polyphenylenethern wird ausführlich in den DE-OSS 32 24 692, 32 24 691 und 33 13 864 sowie den darin aufgeführten Literaturstellen beschrieben.

Mit Beginn der Reaktion wird dem Reaktor Reaktionslösung entnommen. Vorzugsweise erfolgt die Entnahme kontinuierlich. Zwar ist es zu Beginn der Reaktion auch möglich, in kurzzeitigen Intervallen die Reaktionslösung zu entnehmen, aber gegen Ende der Reaktion verändert sich der J-Wert so - schnell, daß es zur gezielten Abstoppung der Reaktion erforderlich ist, kontinuierlich die Reaktionslösung zu entnehmen und die Viskosität zu verfolgen. Die Messung der Viskosität muß bei einer bekannten Temperatur durchgeführt werden. Vorzugsweise handelt es sich um eine konstante Temperatur, die im Bereich der Reaktionstemperatur liegt. Es bietet sich daher an, die dem Reaktor entnommene Lösung in einem Wärmeaustauscher zu thermostatisieren und mit dieser Temperatur durch das Couette-Viskosimeter fließen zu lassen, das ebenfalls auf diese Temperatur eingestellt ist. Der Aufbau eines Couette-Viskosimeters ist beispielsweise Ullmanns Encyklopädie der technischen Chemie Band 5, Seite 765 ff. (1980) zu entnehmen.

Die Reaktionslösung wird sodann in den Reaktor zurückgeführt. Vorzugsweise erfolgt also die Messung der Viskosität in einem Viskosimeter, das von der Flüssigkeit im by-pass durchflossen wird. Die Strömungsgeschwindigkeit wird in bekannter Weise vom Fachmann so eingestellt, daß die axiale Durchströmung durch das Rheometer die Viskositätsmessung nicht wesentlich beeinflußt. Aus dem Viskositätswert läßt sich mit Hilfe einer zuvor aufgenommenen Eichkurve der J-Wert bestimmen. Prinzipiell ist es auch möglich, den J-Wert auf andere Weise zu bestimmen, beispielsweise mit Hilfe einer mathematischen Näherungsformel.

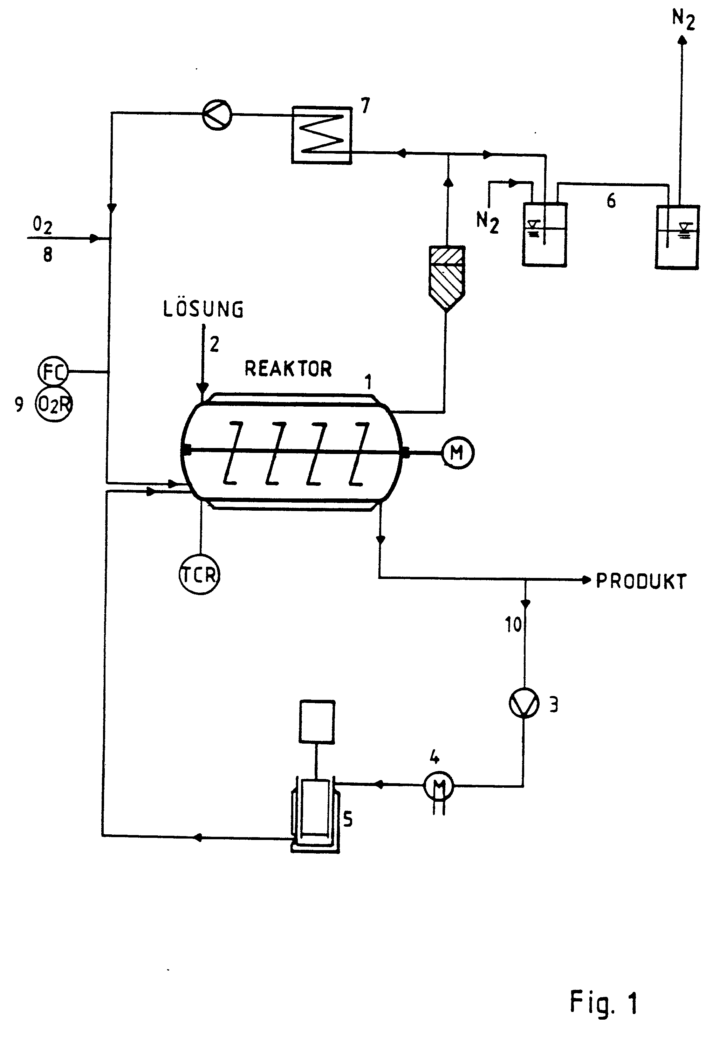

Beispiel: (Figur 1)

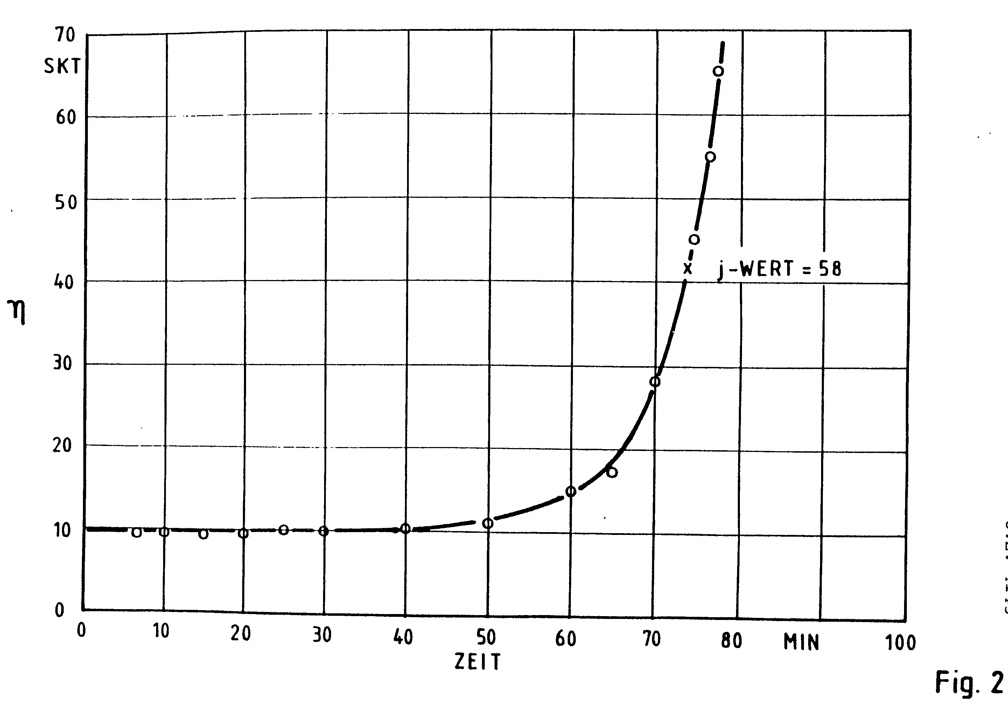

Einem mit einem Rührwerk ausgestatteten thermostabilisierten Reaktor 1, der zu ca. 90 % mit Reaktionslösung gefüllt ist, wird kontinuierlich Sauerstoff zugeführt (vgl. Beispiel 2 der DE-OS 33 13 864). Zur Kontrolle des $O_2$-Gehaltes des Gases im Reaktoreingang wird ein Teilstrom dem Reaktor entnommen und über einen Kreislauf 7 einem Meßgerät 9 zugeführt. Das Abtauchungssystem 6 dient der Druckkontrolle. Die Messung des Fortschrittes der Polykondensation erfolgt mit Hilfe des Kreislaufes 10, der aus einer Pumpe 3, einem Wärmetauscher 4 und einem Couette-Rheometer 5 besteht. Das Rheometer mißt kontinuierlich die Viskosität der Lösung. Der Viskositätsverlauf ist in FIG. 2 dargestellt. Nach 74 Minuten, als der angestrebte J-Wert von 58 erreicht ist, der 42 Skalenteilen (Skt) der Anzeige des Rheometers entspricht, wird die Reaktion durch Zugabe eines Überschusses 50 %iger Essigsäure abgestoppt.

## Ansprüche

1. Verfahren zur kontinuierlichen und schnellen Bestimmung des J-Wertes bei der oxidativen Kupplungsreaktion von Phenolen zu Polyphenylenethern,
dadurch gekennzeichnet,
daß man dem Ansatz während der Reaktion Reaktionslösung einer bekannten Temperatur entnimmt, in einem Couette-Viskosimeter die Viskosität dieser Lösung mißt und mit Hilfe einer Eichkurve oder auf sonstige Weise den J-Wert bestimmt.

2. Verfahren gemäß Anspruch 1,
dadurch gekennzeichnet,
daß man dem Reaktionsansatz kontinuierlich in einem by-pass Reaktionslösung entnimmt, die Temperatur thermostatisiert, in einem Couette-Viskosimeter die Viskosität mißt und den J-Wert daraus bestimmt.

O.Z. 4145

N2
7
6
N2
O2
8
LÖSUNG
2
REAKTOR
1
FC
9
O2R
M
TCR
PRODUKT
10
3
4
M
5

Fig. 1

O.Z. 4145

Fig. 2

j-WERT = 58

η SKT

ZEIT MIN

Europäisches Patentamt

# EUROPÄISCHER RECHERCHENBERICHT

Nummer der Anmeldung

EP 87 10 2799

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| Y,D | EP-A-0 144 106 (ENICHIMICA S.P.A.) * Zusammenfassung; Seite 1, Zeilen 1-3, 12-17; Seite 3, Zeilen 1-9; Seite 4, Zeilen 17-22, Zeile 31 - Seite 5, Zeile 4; Zeilen 30 - Seite 6, Zeile 1; Zeilen 9-12; Ansprüche * --- | 1,2 | G 01 N 11/14 |
| Y | GB-A-2 123 966 (RHEOMETRICS INC.) * Figuren; Seite 1, Zeilen 8-39, 51-54; Zeile 123 - Seite 2, Zeilen 8, 54-58, 88-96; Zeile 116 - Seite 3, Zeilen 1, 42-54 * --- | 1,2 | |
| Y | EP-A-0 122 394 (CHEMISCHE WERKE HÜLS AG) * * Ansprüche * DE - A - 3 313 864 (Kat. D) * --- | 1,2 | RECHERCHIERTE SACHGEBIETE (Int. Cl.4) |
| Y | LABO, Nr. 55, Januar 1974, Seiten 24-26; "Die Viskosität im Seitenstrom" * ganzes Dokument * --- | 1,2 | G 01 N 11/00<br>C 08 G 65/00 |
| A | GB-A-1 197 476 (BRITISH PETROLEUM CO. LTD.) * Seite 1, Zeilen 33-40; Seite 2, Zeilen 46-60; Figuren 1, 2 * --- -/- | 1,2 | |

Der vorliegende Recherchenbericht wurde für alle Patentansprüche erstellt.

| Recherchenort | Abschlußdatum der Recherche | Prüfer |
|---|---|---|
| BERLIN | 29-06-1987 | VINSOME R M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

EP 87 10 2799

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| A | AU-B- 416 045 (TEXACO DEVELOPMENT CORP.)<br>* ganzes Dokument *<br>--- | 1,2 | |
| A | US-A-3 347 089 (C.C. PERRY)<br>* ganzes Dokument *<br>--- | 1,2 | |
| A | DE-B-2 314 671 (H. RUMPF)<br>----- | | |
| | | | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| BERLIN | 29-06-1987 | VINSOME R M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82